(19)

Europäisches
Patentamt
European
Patent Office
Office européen
des brevets

(11)     **EP 1 713 031 B1**

(12)                          **EUROPEAN PATENT SPECIFICATION**

(45) Date of publication and mention
of the grant of the patent:
**10.12.2008   Bulletin 2008/50**

(51) Int Cl.:
***G06T 5/00*** *(2006.01)*

(21) Application number: **06111421.1**

(22) Date of filing: **21.03.2006**

(54) **Method of suppressing a periodical pattern in an image**

Verfahren zur Unterdrückung eines periodischen Musters in einem Bild

Procédé pour supprimer un motif périodique dans une image

(84) Designated Contracting States:
**BE DE FR GB NL**

(30) Priority:   **14.04.2005   EP 05102949**

(43) Date of publication of application:
**18.10.2006   Bulletin 2006/42**

(73) Proprietor: **Agfa HealthCare NV**
**2640 Mortsel (BE)**

(72) Inventors:
• **Behiels, Gert**
**AGFA-GEVAERT**
**2640 Mortsel (BE)**
• **Vuylsteke, Pieter**
**AGFA-GEVAERT**
**2640 Mortsel (BE)**

(74) Representative: **Theunis, Patrick et al**
**Agfa-Gevaert N.V.**
**HE / Intellectual Property 3802**
**Septestraat 27**
**2640 Mortsel (BE)**

(56) References cited:
**EP-A- 1 265 194**          **US-A1- 2003 091 243**

• **CHAU L P ET AL: "DIRECT FORMULATION FOR THE REALIZATION OF DISCRETE COSINE TRANSFORM USING RECURSIVE STRUCTURE" IEEE TRANSACTIONS ON CIRCUITS AND SYSTEMS II: ANALOG AND DIGITAL SIGNAL PROCESSING, IEEE INC. NEW YORK, US, vol. 42, no. 1, January 1995 (1995-01), pages 50-52, XP000495397 ISSN: 1057-7130**

**Description**

FIELD OF THE INVENTION

The present invention relates to a method for suppression of a periodical pattern with a given frequency. It relates more specifically to suppression of periodical patterns caused by the presence of an anti-scatter grid in X-ray images.

BACKGROUND OF THE INVENTION

A commonly used technique to reduce the amount of scattered X-rays in computed radiography, digital radiography as well as classical film-based X-ray systems is the use of anti-scatter grids. These grids are lead foil strips, placed apart at a certain distance in a suitable covering.

There exist different types of anti-scatter grids. In parallel grids, the lead foil strips are parallel, while in honeycomb grids the strips are placed in a honeycomb pattern. The grids are stationary or moving. The use of these grids effectively reduces the radiation scattering but occasionally introduces artefacts such as grid lines into the image.

In a moving system, the motion of the grids removes the grid lines in the image. However, in some circumstances e.g. short exposure time or malfunctioning of the system, the artefacts remain in the image.

If the image is formed digitally or converted afterwards to a digital image representation, Moiré artefacts may appear when displaying the image at a certain scale. These low frequent Moiré artefacts are mostly disturbing and should be eliminated. Before displaying the image, the grid lines, if present in the image, should be removed.

It is a goal of the present invention to eliminate of the negative influence of periodic artefacts e.g. moiré artefacts of zoomed images, without removing diagnostic information.

EP-A-1 265 194 discloses a method comprising the steps of extracting from an image signal the spatial frequency component corresponding to a periodical pattern included therein by applying a transformation, processing the representation of the spatial frequency component, computing the inverse of the transformation and suppressing the spatial frequency component by eliminating from said image signal the result of said inverse transformation.

SUMMARY OF THE INVENTION

The above-mentioned aspects are realised by a method as set out in claim 1.

Specific features for preferred embodiments of the invention are set out in the dependent claims.

The present invention provides a method for suppressing periodical variations centred on given frequency in an image in a given direction, parallel to an image axis.

The method provides a technique to apply a non-linear high frequency attenuating filter parallel and perpendicular to the periodical variation.

This non-linear high frequency attenuating filter can be a median filter. This filter ensures that a minimal amount of relevant information is removed from the original image. This filter does not reduce the image quality of the image.

The method allows the removal of periodic patterns for which the frequency of the variation is not exactly known. It performs a short-time Goertzel transform to obtain a suitable representation of the periodic variation. The result of the short-time Goertzel transform can be presented as a real or imaginary part. A non-linear high frequency attenuating filtering process is performed on this representation or a derivative of this representation. After filtering, removing the diagnostic content from the short-time Goertzel transform, the inverse of the transform is computed and the inverse is used to correct the input data.

The embodiments of the methods of the present invention are generally implemented in the form of a computer program product adapted to carry out the method steps of the present invention when run on a computer. The computer program product is commonly stored in a computer readable carrier medium such as a CD-ROM or a DVD. Alternatively the computer program product takes the form of an electric signal and can be communicated to a user through electronic communication.

BRIEF DESCRIPTION OF THE DRAWINGS

Fig. 1 shows a flowchart for conversion of the multiplicative problem to an additive problem,
Fig. 2 shows an implementation of the invention with a 2-dimensional median filter,
Fig. 3 shows an implementation of the invention with 2 separable median filters,
Fig. 4 shows an implementation of the invention with a highpass filter as a preprocessing step,

Fig. 5 shows the result of the invention on a computer generated input signal. The result of the invention is compared with the result of a similar technique where 3 spectral components in the Fourier domain are suppressed,

Fig. 6 shows the window that is used to compute the short-time Goertzel transform for a signal with a period of 3.011 that needs to be suppressed (see also fig. 5),

Fig. 7 shows the result of the short-time Goertzel transform of the input signal of fig. 5,

Fig. 8 shows the result of the invention on a computer generated input signal. The result of the invention is compared with the result of a similar technique where 3 spectral components in the Fourier domain are suppressed. In this case, the period of the disturbing signal does not coincide with the central frequency of a spectral component in the Fourier domain,

Fig. 9 shows the result of the invention on a computer generated input signal. The result of the invention is compared with the result of a similar technique where 7 spectral components in the Fourier domain are suppressed. In this case, the period of the disturbing signal does not coincide with the central frequency of a spectral component in the Fourier domain,

Fig. 10 shows the result of the invention on a computer generated input signal. The result of the invention is compared with the result of a similar technique where 7 spectral components in the Fourier domain are suppressed. In this case, the period of the disturbing signal varies between 2.911 and 3.111.

Fig. 11 shows the result of the short-time Goertzel transform of the input signal of fig. 10 and the Fourier transform of the said input signal. The output of the short-time Goertzel transform is slowly varying.

Fig. 12 shows a unit step function disturbed with a linear combination of a sine and cosine function of a given frequency.

Fig. 13 shows an amplitude representation of the method of the present invention combined with a non linear postprocessing method, a median filter.

Fig. 14 shows the result of a notch filter for the signal of Fig. 12 and the result of the correction with the method of the present invention, using a non linear postprocessing step presented in Fig. 13.

## DETAILED DESCRIPTION OF THE INVENTION

The presence of an anti-scatter grid manifests itself as a periodic disturbance of the input signal. In the method according to the present invention, we assume that the disturbance is of a pure periodic nature.

It is a goal of the method of the present invention to eliminate of the negative influence of periodic artefacts e.g. moiré artefacts of zoomed images, without removing diagnostic information.

Experiments have shown that removal of sinusoidal signals with the main frequency of the periodic disturbance is good enough for this purpose.

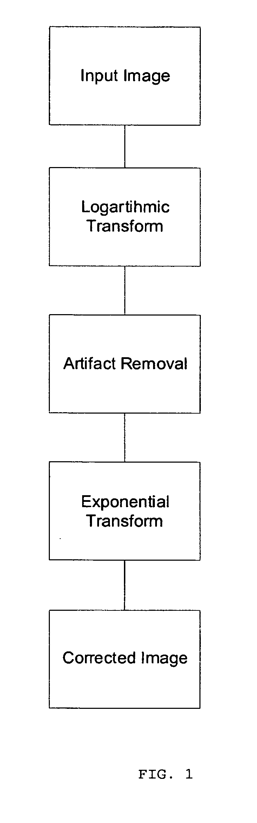

The invention can be used to correct periodic artefacts of an additive or multiplicative nature. In the second case, the situation can be converted to artefacts of an additive nature by using a logarithmic transform before processing and an exponential transform after removal of the periodic artefacts (see figure 1).

The invention comprises the step of processing the image by applying to the image comprising the artefacts a median or high frequency attenuating filter in the direction of the periodic signal, after estimation/extraction of the periodic artefact, to remove the diagnostic information still present in the periodic signal. Normally such a high frequency attenuating filter removes the periodic information of the signal.

In patent application EP 1 598 779 a technique has been described to overcome this problem. This technique only works if the period of the variation is integer.

Because this is not the case for the periodic disturbances of the anti scatter grids, the periodic signal is transformed to a complex representation or amplitude/phase representation of a given frequency.

Such a technique is generally known as the Goertzel transform.

If $s = (s_0, s_1, ..., s_N)$ is the input signal, the Goertzel transform for the frequency $\omega$ is defined as

$$G(\omega) = \sum_{x=0}^{N} s_x \exp(-i\omega x)$$

.

$$G(\omega) = \int s(x) e^{-i\omega x} dx$$

For sake of simplicity, I will only use the integral notation for the formulas. The formulas can be easily converted to our discrete problem. For suppression of the periodical patterns caused by the presence of an anti-scatter grid in X-ray images, the frequency $\omega$ however, is only known to a certain degree of accuracy. Also, because of the nature of the anti scatter grids, this frequency can change a little bit over distance. According to the invention a windowed Goertzel transform, localized in place, is computed. This transform will be called the short-time Goertzel transform $\Gamma$

### Equation 1

$$\Gamma(y, \omega) = \int w(x - y)s(x)e^{-i\omega x}dx$$

The result of Equation 1 for a given frequency $\omega$, is a complex number for each position y. The inverse transform of the complex number is a good estimate of the periodic disturbance.

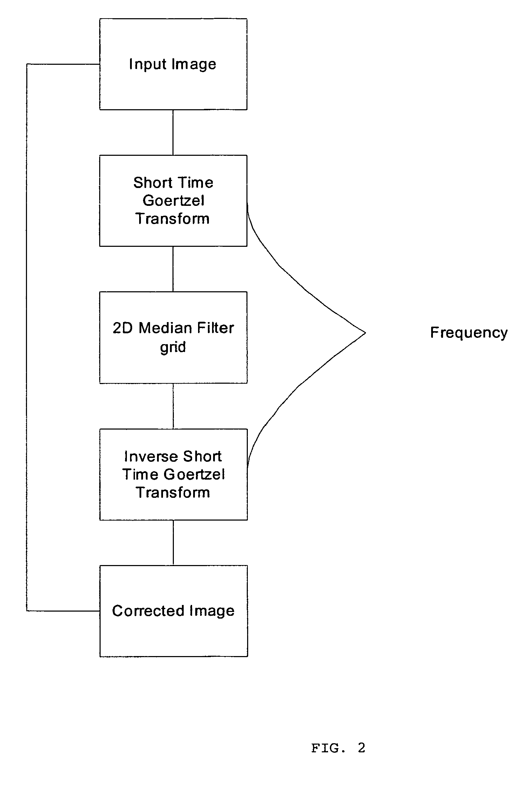

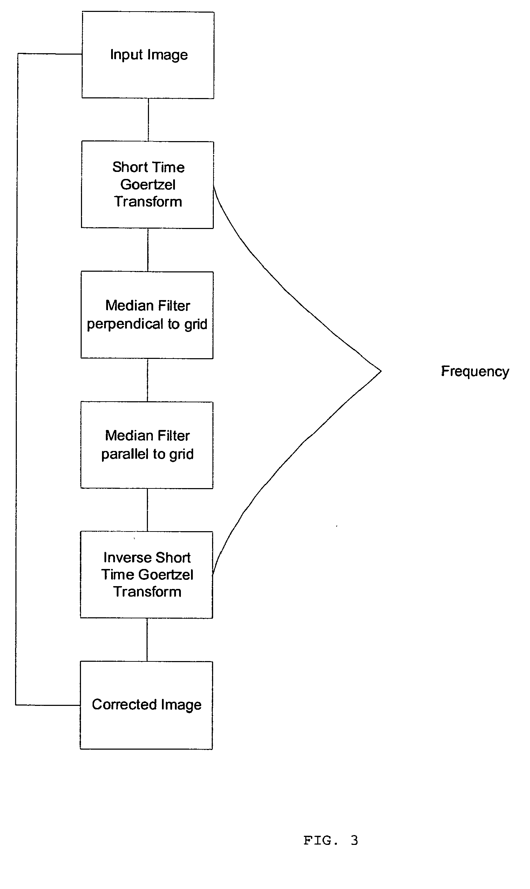

However, we perform a median or high frequency attenuating filter on this complex representation (or any part or derivative of this representation e.g.: real part, imaginary part, amplitude, phase ...). This filter may consist of a 2-dimensional filter, or two separate filters, one parallel to the grid direction and one orthogonal to the grids (see figure 2 and figure 3).

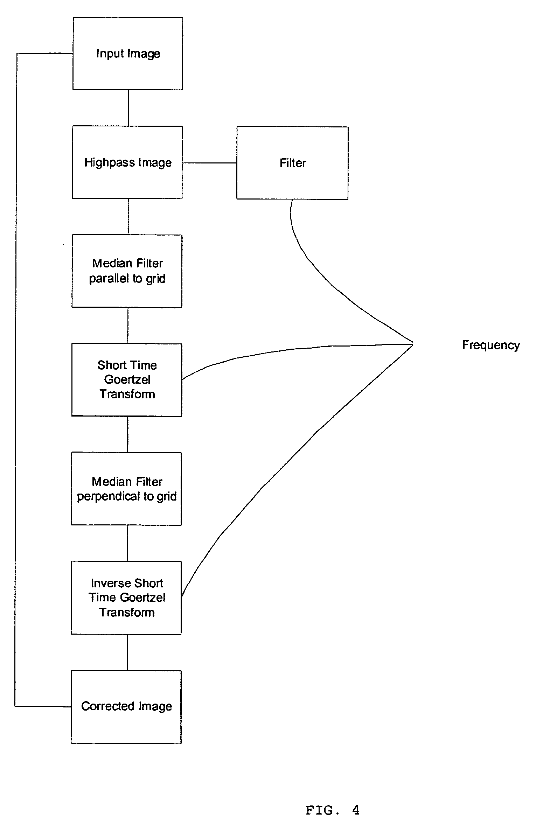

If we follow a scheme where we already have applied a high frequency attenuating filter parallel to the grids, only a filter orthogonal to the grids is sufficient for robust estimation of the periodic signal (Figure 4).

If we assume that the input signal s can be defined as

$$s(x) = \alpha(x)\cos(\omega x) + \beta(x)\sin(\omega x),$$

which is a reasonable assumption for a model of an antiscatter grid. We want to extract the slowly changing amplitudes $\alpha, \beta$ of the grid We divide Equation 1 into the following two parts:

$$\Gamma_c(y, \omega) = \int w(x - y)s(x)\cos(x)dx$$

$$\Gamma_s(y, \omega) = \int w(x - y)s(x)\sin(x)dx$$

Equation 1 transforms to $\Gamma(y, \omega) = \Gamma_c(y, \omega) + i\Gamma_s(y, \omega)$. If we assume that the amplitudes are approximately constant over the extent of our window, we can use the following notations:

$$\Gamma_c(y, \omega) \approx \alpha(y)\Gamma_{cc}(y, \omega) + \beta(y)\Gamma_{cs}(y, \omega)$$

$$\Gamma_s(y, \omega) \approx \alpha(y)\Gamma_{cs}(y, \omega) + \beta(y)\Gamma_{ss}(y, \omega)$$

with

$$\Gamma_{cc}(y, \omega) = \int w(x - y)\cos(x)\cos(x)dx$$

$$\Gamma_{cs}(y, \omega) = \int w(x - y)\cos(x)\sin(x)dx \quad .$$

$$\Gamma_{scc}(y, \omega) = \int w(x - y)\cos(x)\cos(x)dx$$

With this knowledge, we can approximate the amplitudes

Equation 2

$$\alpha(y) = \frac{\Gamma_c(y,\omega)\Gamma_{ss}(y,\omega) + \Gamma_s(y,\omega)\Gamma_{cs}(y,\omega)}{\Gamma_{cc}(y,\omega)\Gamma_{ss}(y,\omega) - \Gamma_{cs}(y,\omega)\Gamma_{cs}(y,\omega)}$$

$$\beta(y) = \frac{\Gamma_c(y,\omega)\Gamma_{sc}(y,\omega) - \Gamma_s(y,\omega)\Gamma_{cc}(y,\omega)}{\Gamma_{cc}(y,\omega)\Gamma_{ss}(y,\omega) - \Gamma_{cs}(y,\omega)\Gamma_{cs}(y,\omega)}$$

If some postprocessing on the amplitude representation is performed, resulting in the new amplitude representation $(\alpha',\beta')$ the reconstructed signal s' is given by

$$s^{'}(x) = \alpha'(x)\cos(\omega x) + \beta'(x\_\sin(\omega x)$$

The advantages of this transformation are twofold. Only one spectral component needs to be computed and the transformation generates a slowly varying output, which is ideal for processing before transforming it back to the original input domain.

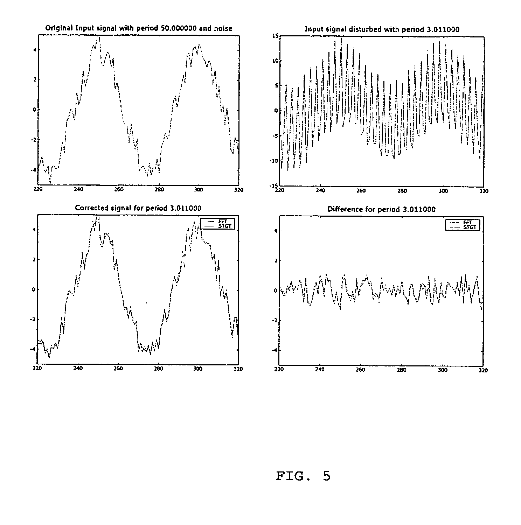

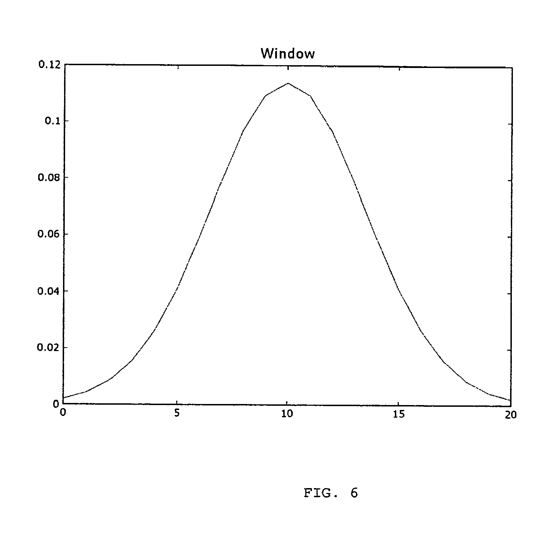

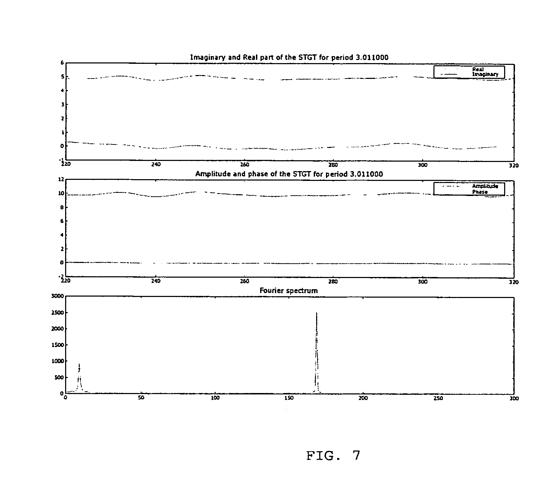

For an input signal with a period of 50 with a little bit of noise, which is disturbed by a synthetic grid with a period of 3.011 pixels (figure 5), we compare our approach with an approach in which 3 spectral components in the Fourier domain are suppressed. The window that is used to compute the short-time Goertzel transform for the frequency

$$\omega = \frac{2\pi}{3.011}$$

is displayed in figure 6. In figure 7 the output of the short-time Goertzel transform is displayed as both parts of the complex notation and as an amplitude-phase notation. Both representations vary smoothly, which makes it easy to apply high frequency attenuating filters to remove remaining diagnostic information. Figure 7 also displays the Fourier spectrum of the input signal.

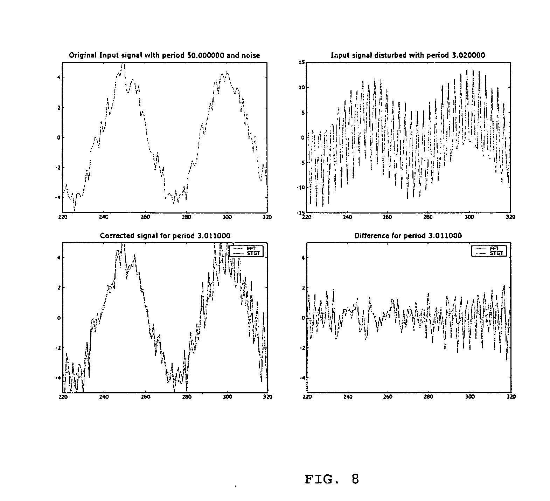

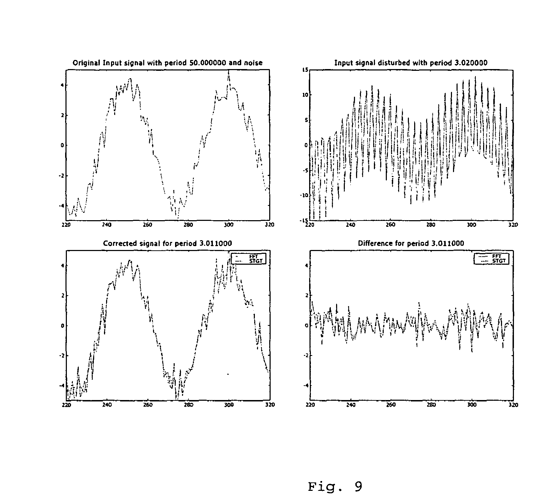

In the assumption that the period of the signal is not 3.011 but 3.02, the outer limit of the same frequency bin in the Fourier domain, we have different outcomes (see figure 8). The short-time Goertzel transform performs better than the Fourier method. We can achieve the same result with the Fourier technique, but than we need to suppress more spectral components. In figure 9 seven spectral components are suppressed. This makes the Fourier transform more time consuming.

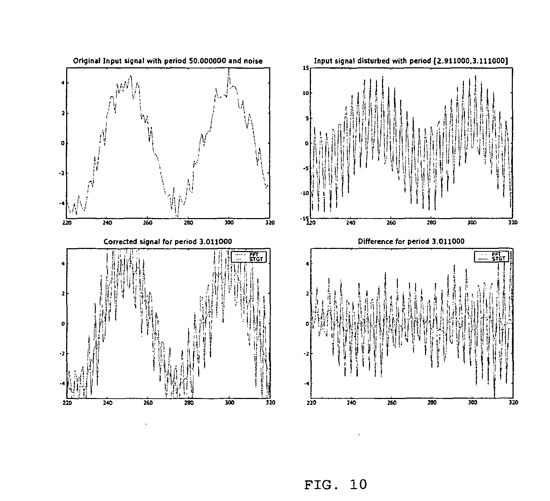

The method is even more interesting when we take a signal in which the period changes. In figure 10 an input signal is generated where the period of the grid varies from 2,911 to 3.111 pixels. By suppressing 7 spectral components, we do not have the same result as with our short-time Goertzel transform.

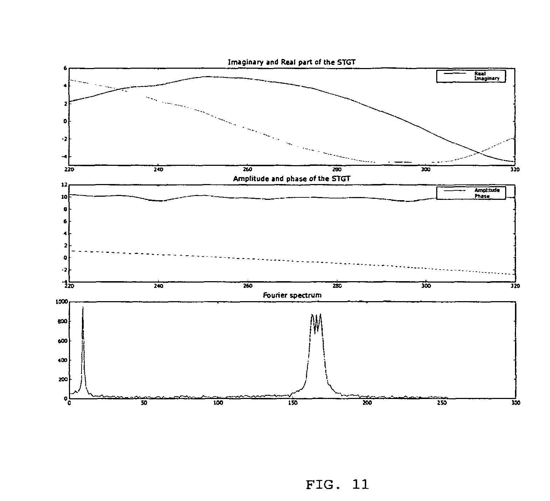

An alternative to the Goertzel transform is to design a notch filter which has the same filtering characteristics than the short-time Goertzel transform. The Goertzel transform however, has the advantage that high frequency attenuation, spike-detection algorithms, are easier to implement in the Goertzel domain since its output is slowly varying (see figure 7 and figure 11).

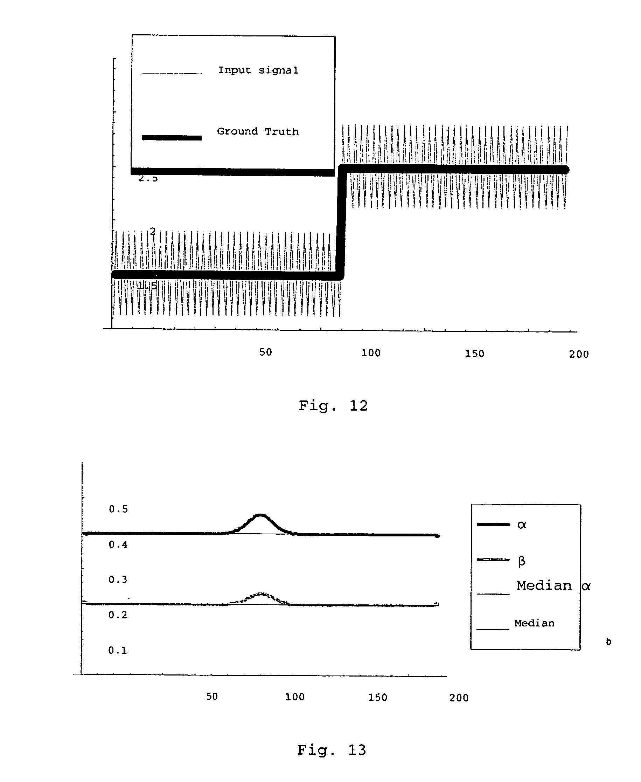

The difference between a notch filter and the short time Goertzel transform combined with a non linear filter, in this case a median filter for the amplitude representation of Equation 2, is illustrated on the input signal of figure 12. The input signal consists of a unit step disturbed with a signal of a given frequency:

$$s(x) = 1.5 + UnitStep(x - 128) + 0.4\cos(\omega x) + 0.2\sin(\omega x)$$

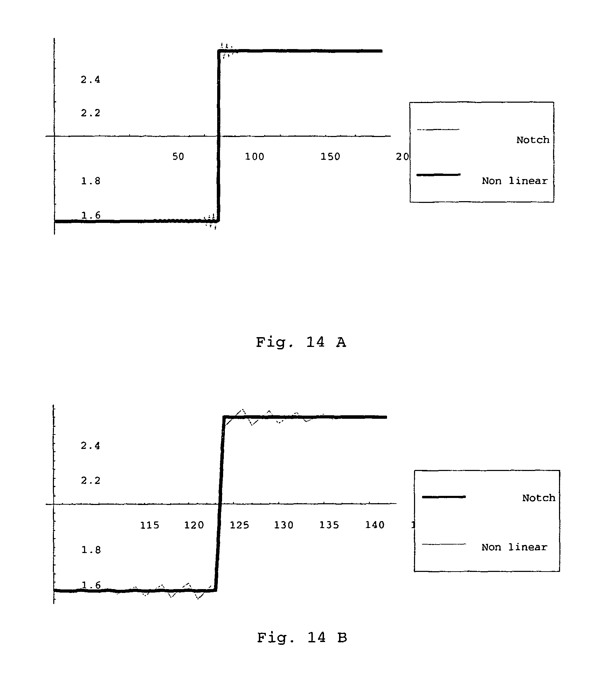

In figure 13, the amplitude representation $(\alpha,\beta)$ is shown together with a median filter $(\alpha',\beta')$ version of this amplitude representation. The difference between our non linear short time goertzel transform and a notch filter is given in figure 14. We clearly see that at that our filter is capable to almost perfectly reconstruct the step function. The small fluctuations, due to rounding errors, are much smaller than the fluctuations introduced by notch filter.

**Claims**

1.  A periodical pattern suppression method wherein the spatial frequency component corresponding to a periodical pattern occurring in an image signal is suppressed, comprising the steps of

    - extracting from said image signal the spatial frequency component corresponding to a periodical pattern included therein by applying a transformation to the image signal to transform it into a representation of the spatial frequency component,
    - processing the representation of the spatial frequency component,
    - computing the inverse of the transformation on the processed representation of the spatial frequency component,
    - suppressing the spatial frequency component occurring in the image signal by eliminating from said image signal the result of said inverse transformation **characterized in that** said transformation is a short-time Goertzel transform defined by the formula

    $$\Gamma(y, \omega) = \int w(x - y)s(x)e^{-i\omega x}dx$$

    wherein s(x) is said image signal, w defines weights, w represents the given frequency of the periodical pattern and y designates a position.

2.  A method according to claim 1 wherein said processing comprises applying a non-linear high frequency attenuating filter to the representation of the spatial frequency component.

3.  A method according to claim 2 wherein the high frequency attenuating filter is implemented with a median filter.

4.  A method according to claim 2 where the high frequency attenuating filter is a 2-dimensional filter.

5.  A method according to claim 2 wherein the high frequency attenuating filter is a 1-dimensional filter in the same direction as the spatial frequency component.

6.  A method according to claim 2 where the high frequency attenuation filter is a 1-dimensional filter in the orthogonal direction to the spatial frequency component.

7.  A method according to claim 2 where the high frequency attenuating filter is a sequence of high frequency attenuating filters, both 1-dimensional or two-dimensional.

8.  A method according to claim 2 where the filter is applied to the imaginary part of the complex representation.

9.  A method according to claim 2 where the filter is applied to the real part of the complex representation.

10. A method according to claim 2 where the filter is applied to the magnitude of the complex representation.

11. A method according to claim 2 where the filter is applied to the phase of the complex representation.

12. A method according to any of the preceding claims wherein the transformation is applied to a filtered version of the image signal.

13. A method according to claim 12 where the filtered version of the image signal does not contain the DC component.

14. A method according to claim 12 where the filtered version of the image signal only contains frequencies within a certain bandwidth centred on a frequency to be suppressed.

15. A method according to claim 1 wherein the spatial frequency components are removed which correspond to an image of anti-scatter gridlines in a radiographic image.

16. A computer program product comprising computer code for performing the steps of any of the preceding claims when run on a computer.

17. A computer readable carrier medium comprising computer executable program code adapted to carry out the steps of any of claims 1 to 15.

**Patentansprüche**

1. Ein Verfahren zum Löschen eines periodischen Musters, wobei in diesem Verfahren die Ortsfrequenzkomponente, die einem in einem Bildsignal vorkommenden periodischen Muster entspricht, gelöscht wird, wobei das Verfahren durch folgende Schritte **gekennzeichnet** ist :

   - Extrahieren aus dem Bildsignal von der einem im Bildsignal vorkommenden periodischen Muster entsprechenden Ortsfrequenzkomponente, indem auf das Bildsignal eine Transformation durchgeführt wird, um es in eine Darstellung der Ortsfrequenzkomponente umzuwandeln,
   - Verarbeitung der Darstellung der Ortsfrequenzkomponente,
   - Berechnung der Umkehrfunktion der Transformation auf der verarbeiteten Darstellung der Ortsfrequenzkomponente,
   - Löschen der im Bildsignal vorkommenden Ortsfrequenzkomponente, indem aus dem Bildsignal das Ergebnis der Umkehrtransformation gelöscht wird, **dadurch gekennzeichnet, dass** die Transformation eine kurzzeitige Goertzel-Transformation nach folgender Formel ist :

$$\Gamma(y, \omega) = \int w(x - y)s(x)e^{-i\omega x}dx$$

   in der s(x) das Bildsignal bedeutet, w Gewichte definiert, ω die vorgegebene Frequenz des periodischen Musters bedeutet und y eine Position bedeutet.

2. Verfahren nach Anspruch 1, **dadurch gekennzeichnet, dass** die Verarbeitung die Anwendung eines nicht-linearen Hochfrequenz-Dämpfungsfilters auf die Darstellung der Ortsfrequenzkomponente umfasst.

3. Verfahren nach Anspruch 2, **dadurch gekennzeichnet, dass** der Hochfrequenz-Dämpfungsfilter mit einem Medianfilter ausgestattet ist.

4. Verfahren nach Anspruch 2, **dadurch gekennzeichnet, dass** der Hochfrequenz-Dämpfungsfilter mit einem zweidimensionalen Filter ausgestattet ist.

5. Verfahren nach Anspruch 2, **dadurch gekennzeichnet, dass** der Hochfrequenz-Dämpfungsfilter ein eindimensionaler Filter ist, der in die gleiche Richtung wie die Ortsfrequenzkomponente filtert.

6. Verfahren nach Anspruch 2, **dadurch gekennzeichnet, dass** der Hochfrequenz-Dämpfungsfilter ein eindimensionaler Filter ist, der orthogonal zur Ortsfrequenzkomponente filtert.

7. Verfahren nach Anspruch 2, **dadurch gekennzeichnet, dass** der Hochfrequenz-Dämpfungsfilter eine Serie von sowohl eindimensionalen als zweidimensionalen Hochfrequenz-Dämpfungsfiltern ist.

8. Verfahren nach Anspruch 2, **dadurch gekennzeichnet, dass** der Filter auf den imaginären Teil der komplexen Darstellung angewandt wird.

9. Verfahren nach Anspruch 2, **dadurch gekennzeichnet, dass** der Filter auf den Realteil der komplexen Darstellung angewandt wird.

10. Verfahren nach Anspruch 2, **dadurch gekennzeichnet, dass** der Filter auf die Größe der komplexen Darstellung angewandt wird.

11. Verfahren nach Anspruch 2, **dadurch gekennzeichnet, dass** der Filter auf die Phase der komplexen Darstellung angewandt wird.

**12.** Verfahren nach einem der vorstehenden Ansprüche, **dadurch gekennzeichnet, dass** die Transformation auf eine gefilterte Version des Bildsignals angewandt wird.

**13.** Verfahren nach Anspruch 12, **dadurch gekennzeichnet, dass** die gefilterte Version des Bildsignals die Gleichstromkomponente nicht enthält.

**14.** Verfahren nach Anspruch 12, **dadurch gekennzeichnet, dass** die gefilterte Version des Bildsignals nur Frequenzen innerhalb einer vorgegebenen, auf eine zu löschende Frequenz zentrierten Bandbreite enthält.

**15.** Verfahren nach Anspruch 1, **dadurch gekennzeichnet, dass** diejenigen Ortsfrequenzkomponenten, die einem Bild von Streustrahlenrasterlinien in einem Röntgenbild entsprechen, ausgesondert werden.

**16.** Ein Computerprogrammprodukt, enthaltend einen Programmcode, der bei Verwendung des Programms auf einem Computer die Ausführung der Schritte nach einem der vorstehenden Ansprüche sichert.

**17.** Ein computerlesbares Trägermedium, das einen durch einen Computer ausführbaren Code enthält, der die Ausführung der Schritte nach einem der Ansprüche 1 bis 15 sichert.

**Revendications**

**1.** Procédé de suppression du motif périodique, dans lequel on supprime la composante de fréquence spatiale correspondant à un motif périodique apparaissant dans un signal d'image, comprenant les étapes consistant à :

- extraire dudit signal d'image, la composante de fréquence spatiale correspondant à un motif périodique qui y est incluse en appliquant une transformation sur le signal d'image pour le transformer en une représentation de la composante de fréquence spatiale ;
- traiter la représentation de la composante de fréquence spatiale ;
- calculer l'inverse de la transformation sur la représentation traitée de la composante de fréquence spatiale ;
- supprimer la composante de fréquence spatiale apparaissant dans le signal d'image en éliminant dudit signal d'image le résultat de ladite transformation inverse, **caractérisé en ce que** ladite transformation est une transformée de Goertzel à court terme définie par la formule :

$$\Gamma(y, \omega) = \int w(x - y)s(x)e^{-i\omega x}dx$$

dans laquelle s(x) représente ledit signal d'image, w définit des pondérations, ω représente la fréquence donnée du motif périodique et y désigne une position.

**2.** Procédé selon la revendication 1, dans lequel ledit traitement comprend le fait d'appliquer un filtre d'atténuation des hautes fréquences non linéaires sur la représentation de la composante de fréquence spatiale.

**3.** Procédé selon la revendication 2, dans lequel le filtre d'atténuation des hautes fréquences est mis en oeuvre avec un filtre médian.

**4.** Procédé selon la revendication 2, dans lequel le filtre d'atténuation des hautes fréquences est un filtre bidimensionnel.

**5.** Procédé selon la revendication 2, dans lequel le filtre d'atténuation des hautes fréquences est un filtre unidimensionnel dans la même direction que celle de la composante de fréquence spatiale.

**6.** Procédé selon la revendication 2, dans lequel le filtre d'atténuation des hautes fréquences est un filtre unidimensionnel dans la direction perpendiculaire à celle de la composante de fréquence spatiale.

**7.** Procédé selon la revendication 2, dans lequel le filtre d'atténuation des hautes fréquences est une séquence de filtres d'atténuation des hautes fréquences, à la fois unidimensionnels et bidimensionnels.

**8.** Procédé selon la revendication 2, dans lequel le filtre est appliqué sur la partie imaginaire de la représentation complexe.

**9.** Procédé selon la revendication 2, dans lequel le filtre est appliqué sur la partie réelle de la représentation complexe.

**10.** Procédé selon la revendication 2, dans lequel le filtre est appliqué sur l'ampleur de la représentation complexe.

**11.** Procédé selon la revendication 2, dans lequel le filtre est appliqué sur la phase de la représentation complexe.

**12.** Procédé selon l'une quelconque des revendications précédentes, dans lequel la transformation est appliquée sur une version filtrée du signal d'image.

**13.** Procédé selon la revendication 12, dans lequel la version filtrée du signal d'image ne contient pas la composante de courant continu.

**14.** Procédé selon la revendication 12, dans lequel la version filtrée du signal d'image contient uniquement les fréquences situées au sein d'une certaine bande passante centrée sur une fréquence à supprimer.

**15.** Procédé selon la revendication 1, dans lequel on retire les composantes de fréquence spatiale qui correspondent à une image de grille d'antidiffusion dans une image radiographique.

**16.** Produit de programme informatique comprenant un code informatique pour la mise en oeuvre des étapes selon l'une quelconque des revendications précédentes, lorsqu'il est exécuté sur un ordinateur.

**17.** Support lisible par ordinateur, comprenant un code de programme exécutable par ordinateur pour la mise en oeuvre des étapes selon l'une quelconque des revendications 1 à 15.

FIG. 1

FIG. 2

FIG. 3

FIG. 4

FIG. 5

FIG. 6

FIG. 7

Original Input signal with period 50.000000 and noise

Input signal disturbed with period 3.020000

Corrected signal for period 3.011000

Difference for period 3.011000

FIG. 8

Fig. 9

FIG. 10

FIG. 11

Fig. 12

Fig. 13

Fig. 14 A

Fig. 14 B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1265194 A

- EP 1598779 A